# EUROPÄISCHE PATENTSCHRIFT

(11) **EP 0 009 206 B1**
(45) Veröffentlichungstag und Bekanntmachung des Hinweises auf die Patenterteilung: **14.10.1981**
(21) Anmeldenummer: 79103411.9
(22) Anmeldetag: 12.09.1979
(51) Int. Cl.: C07D 211/90, C07D 401/04, C07D 405/04, C07D 409/04, A61K 31/44

(54) **Fluorhaltige 1,4-Dihydropyridine, diese enthaltende Arzneimittel sowie Verfahren zu ihrer Herstellung**
Fluor-containing 1,4-dihydropyridines, pharmaceuticals containing them and process for their preparation
1,4-Dihydropyridines fluorées, médicaments les contenant et procédé pour leur préparation

(30) Priorität: 25.09.1978 DE 2841667
(43) Veröffentlichungstag der Anmeldung: 02.04.1980
(73) Patentinhaber: BAYER AG, 51368 Leverkusen (DE)
(72) Erfinder: Horstmann, Harald, Dr., D-5600 Wuppertal 1 (DE); Bossert, Friedrich, Dr., D-5600 Wuppertal 1 (DE); Heise, Arend, Dr., D-5600 Wuppertal 11 (DE); Kazda, Stanislav, Dr., D-5600 Wuppertal 1 (DE)

## Beschreibung

Die vorliegende Erfindung betrifft neue 1,4-Dihydropyridinderivate, die in ihren Estergruppen perfluorierte Kohlenstoffatome enthalten, ein Verfahren zu ihrer Herstellung diese enthaltende Arzneimittel insbesondere kreislaufbeeinflussende Mittel, sowie Verfahren zur Herstellung dieser Arzneimittel.

Es ist bereits bekanntgeworden, daß man 1,4-Dihydropyridincarbonsäureester mit interessanten kreislaufbeeinflussenden Eigenschaften erhält durch Umsetzung von Aldehyden mit ß-Ketocarbonsäureestern und Enaminocarbonsäureestern (vgl. DE-OS 2117571 und DE-OS 2117573). Dihydropyridinderivate, die in ihren Estergruppen perfluorierte Kohlenstoffatome enthalten, sind bisher noch nicht beschrieben worden.

Die vorliegende Erfindung betrifft neue 1,4-Dihydropyridine mit mindestens einem perfluorierten Kohlenstoffatom in der Estergruppe der allgemeinen Formel (I) 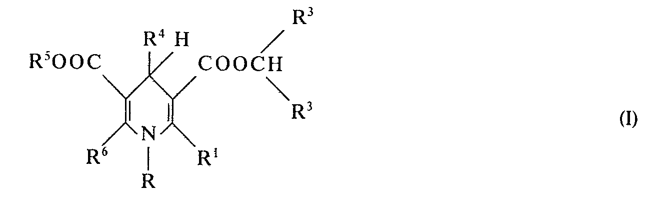 in welcher
R für Wasserstoff, Alkyl mit 1 bis 2 Kohlenstoffatomen oder Benzyl steht,
R' und R⁶ gleich oder verschieden sind und für Alkyl mit 1 bis 4 Kohlenstoffatomen stehen,
R⁴ für Phenyl steht, welches gegebenenfalls durch ein oder zwei Substituenten aus der Gruppe Nitro, Cyano, Trifluormethyl, Halogen, Acido, Trifluormethoxy, Alkyl, Alkoxy, Alkylmercapto oder S0₂-Alkyl mit jeweils 1 bis 2 Kohlenstoffatomen in den Alkyl- und Alkoxyresten substituiert ist oder für einen Pyridylrest steht,
R⁵ für Alkyl mit 1 bis 6 Kohlenstoffatomen, welches gegebenenfalls durch Halogen substituiert ist steht oder für Alkoxyalkyl, Alkenyl, Alkinyl mit bis zu 6 Kohlenstoffatomen steht oder für Aminoalkyl mit 1 bis 4 Kohlenstoffatomen im Alkylrest steht, wobei die beiden Wasserstoffatome der Aminogruppe durch 1 oder 2 Alkylgruppen mit 1 bis 4 Kohlenstoffatomen und/oder durch eine Benzylgruppe ersetzt sein können oder für den Rest 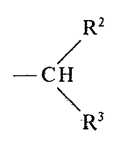 steht, und
R² und R³ jeweils gleich oder verschieden sind, wobei einer der beiden Substituenten Wasserstoff bedeuten kann und der andere Substituent oder beide Substituenten R² und R³ jeweils für einen geradkettigen, verzweigten oder cyclischen Alkylrest mit bis zu 8 Kohlenstoffatomen, vorzugsweise mit bis zu 6 Kohlenstoffatomen, stehen, der 1, 2 oder 3 perfluorierte Kohlenstoffatome mit jeweils 2 oder 3 Fluorsubstituenten enthält, wobei dieser Alkylrest gegebenenfalls durch die Gruppierung -CH₂-X-CH₂- unterbrochen ist, wobei X Sauerstoff oder Schwefel bedeutet oder wobei
R² und R³ gemeinsam mit der CH-Gruppe einen gesättigten isocyclischen Rest mit 4 bis 7 Ringgliedern bilden können, der 1, 2 oder 3 perfluorierte C-Atome enthält und der gegebenenfalls durch eine Alkylgruppe mit 1 bis 4 Kohlenstoffatomen substituiert ist, wobei diese Alkylgruppe ihrerseits wieder ein oder zwei perfluorierte Kohlenstoffatome enthalten kann, sowie ihre pharmazeutisch unbedenklichen Säureadditionssalze.

Es wurde gefunden, daß man die neuen Dihydropyridinderivate der allgemeinen Formel (I) erhält, wenn man 1 Mol Enamin der allgemeinen Formel (II) 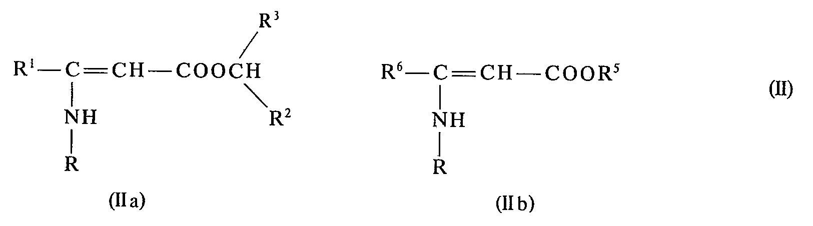 in welcher
R, R^{l}, R², R³, R⁵ und R⁶ die oben angegebene Bedeutung haben,
mit einem Mol β-Ketocarbonsäureester der allgemeinen Formel III 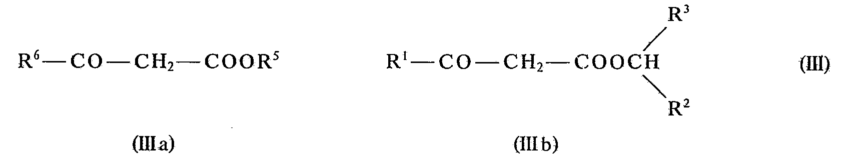 in welcher
R¹, R², R³, R⁵ und R⁶ die oben angegebene Bedeutung haben,
mit einem Mol Aldehyd der allgemeinen Formel (IV) 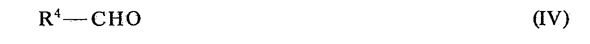 in welcher
R⁴ die oben angegebene Bedeutung besitzt,
gegebenenfalls nach Isolierung der sich aus (111) und (IV) bildenden Ylidenverbindungen der allgemeinen Formel (V) 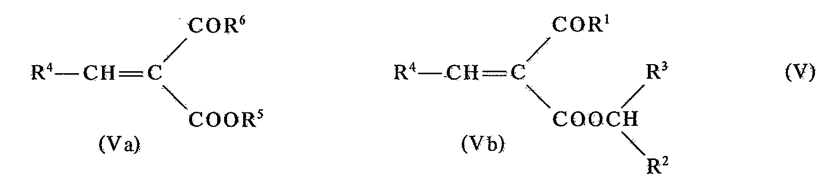 in welcher
R¹, R², R³, R⁴, R⁵ und R⁶ die oben angegebene Bedeutung besitzen,

gegebenenfalls in Gegenwart von Wasser oder inerte organischer Lösungsmittel bei Temperaturen zwischen 20 und 150°C umsetzt.

Für den Fall, daß die erfindungsgemäßen Verbindungen der Formel einen basischen Substituenten tragen, können diese Verbindungen mit geeigneten organischen oder anorganischen Säuren nach an sich bekannten Methoden in erfindungsgemäße, pharmazeutisch unbedenkliche Säureadditionssalze umgewandelt werden.

Die Verbindungen der Formel 1 sowie ihre pharmazeutisch unbedenklichen Säureadditionssalze zeigen starke pharmakologische Wirkungen. Insbesondere zeichnen sie sich durch Wirkungen auf den Kreislauf aus und können vorzugsweise verwendet werden als Coronarmittel, antihypertensive Mittel und Mittel zur Steigerung der peripheren Durchblutung. Aufgrund ihrer neuartigen Struktur, insbesondere aufgrund der Anwesenheit von perfluorierten Kohlenstoffatomen besitzen die erfindungsgemäßen Verbindungen vorteilhafte Wirkungen.

Als Beispiel für die unerwartet vorteilhaften Eigenschaften der erfindungsgemäßen neuen Verbindungen ist die sehr spezifische Erhöhung der Kolateral-Durchblutung von ischämischen Extremitäten anzusehen. Neben dieser starken dilatierenden Wirkung auf die Kolateralen der ischämischen Extremität, zeigen die Verbindungen überraschenderweise nur eine wesentlich schwächere Wirkung auf die normale Durchblutung der intakten Extremität. Sie sind damit besonders geeignet als Arzneimittel zur Behandlung von peripheren Durchblutungsstörungen.

Die unerwartet spezifische Wirkung auf die kolaterale Durchblutung wird beispielhaft durch die Daten der folgenden Tabelle belegt. Hier wurde die Verbindung des Beispiels 13 mit chemisch ähnlichen Verbindungen aus den bekannten Offenlegungsschriften DE-OS 2 117 571, DE-OS 2 117 572 und DE-OS 2 117 573 verglichen. Insbesondere der Vergleich mit der Verbindung des Beispiels 9 aus DE-OS 2 117 573 zeigt, daß die spezifisch Kolateral-Wirkung unerwarteter Weise bedingt wird durch die Einführung von Esterresten mit mindestens einem perfluorierten Kohlenstoffatom. Dieser Befund konnte bei Kenntnis des Standes der Technik nicht erwartet werden.

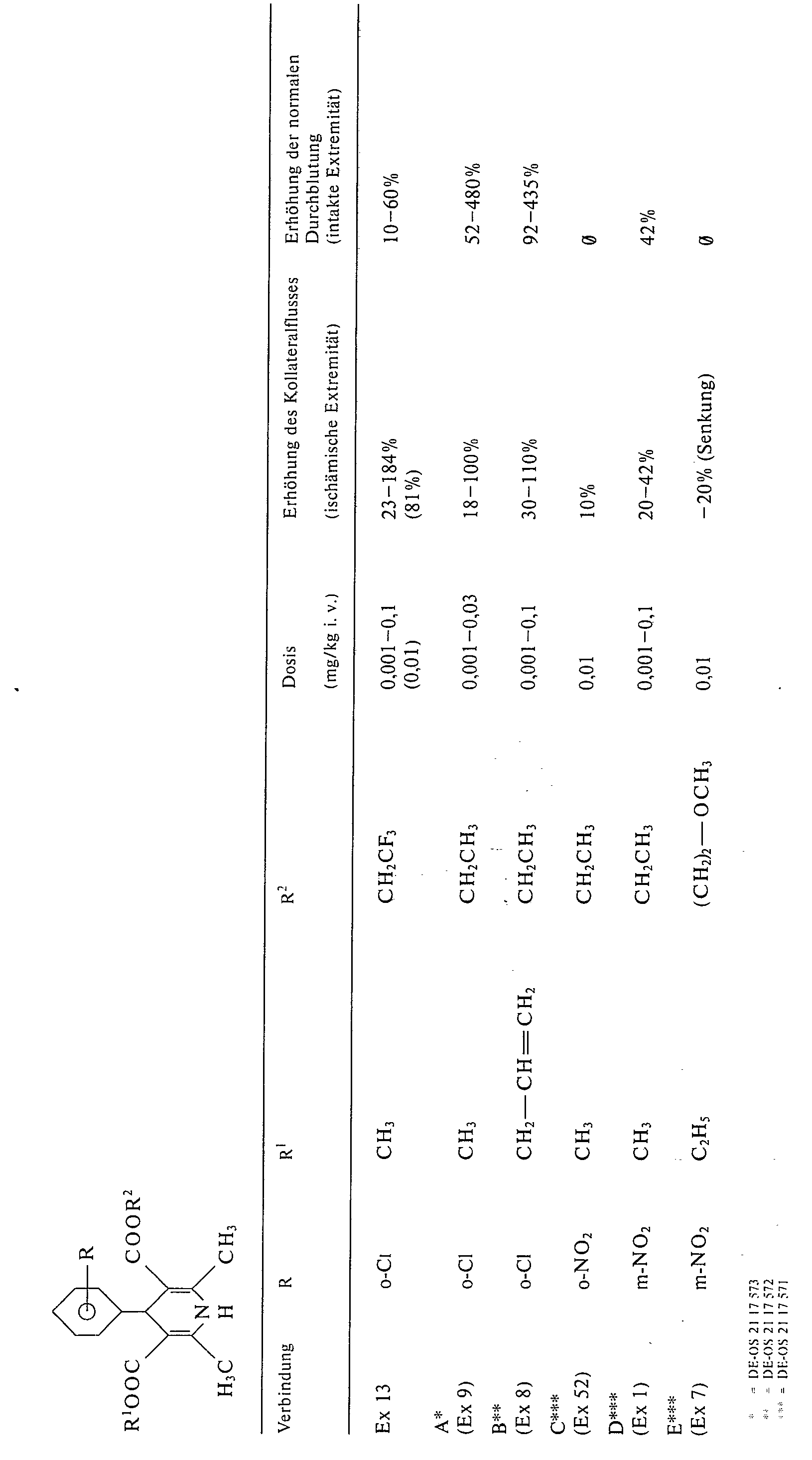

Die erfindungsgemäß verwendbaren Enamine der allgemeinen Formel II sind bekannt oder können nach bekannten Methoden aus den entsprechenden β-Dike o-Verbindungen und Aminen hergestellt werden (vgl. A. Pinner B 34,4239/40 [1901 ]).

Als Beispiele seien genannt:
β-Aminocrotonsäure-2-trifluoräthylester
β-Aminocrotonsäure-2,2-hexafluor-isopropylester
β-Aminocrotonsäure-2-trifluormethylisopropylester
β-Aminocrotonsäure-3,4-penta-fluorbutylester
β-Aminocrotonsäure-4-trifluor-methylcyclohexylester
β-Aminocrotonsäure-2-trifluoräthoxy-äthylester
β-Aminocrotonsäure-methylester
ß-Aminocrotonsäure-äthylester
β-Aminocrotonsäure-isopropylester
β-Aminocrotonsäure-2-benzylaminoäthylester
β-Aminocrotonsäure-2-chloräthylester
β-Aminocrotonsäure-2-methoxymethylester
β-Aminocrotonsäure-cyclohexylester
β-Aminocrotonsäure-allylester
β-Aminocrotonsäure-propargyl-ester

Die erfindungsgemäß verwendbaren β-Ketocarbonsäureester der allgemeinen Formel III sind bekannt oder können nach bekannten Methoden hergestellt werden (vgl. Houben-Weyl, Methoden der organischen Chemie VII/4, 230 ff [(196]).

Als Beispiele seien genannt:
Acetessigsäuremethylester
Acetessigsäureäthylester
Acetessigsäureisopropylester
Acetessigsäurehexylester
Acetessigsäurebenzylester
Acetessigsäurecyclohexylester
Acetessigsäure-2-morpholinoäthylester
Acetessigsäure-2-dimethylamino-äthylester
Acetessigsäure-3-diäthylamino-propylester
Acetessigsäure-2-methoxyäthylester
Acetessigsäure-2-äthoxy-äthylester
Acetessigsäure-2-isopropoxy-äthylester
Acetessigsäure-2-benzylaminoäthylester
Acetessigsäure-2-(α-methylbenzyl)-aminoäthylester
Acetessigsäure-2-(x-methylphenäthyl)-aminoäthylester
Acetessigsäure-trifluoräthylester
Acetessigsäure-2-trifluorisopropylester
Acetessigsäure-2,2-hexafluorisopropylester
Acetessigester-3,4-pentafluorbutylester
Acetessigsäure-3-trifluormethyl-propenylester
Acetessigsäure-4-trifluormethyl-cyclohexylester
Acetessigsäure-2-trifluoräthoxy-äthylester
Acetessigsäure-2-trifluoräthylmercapto-äthylester

Die erfindungsgemäß verwendbaren Aldehyde der Formel IV sind bekannt oder können nach bekannten Methoden hergestellt werden (vgl. E. Mosettig, Org. Reactions VIII, 218 ff [(1954]). Als Beispiele seien genannt:
Acetaldehyd
Benzaldehyd
.o-Nitrobenzaldehyd
m-Nitrobenzaldehyd
o-Fluorbenzaldehyd
o-Chlorbenzaldehyd
m-Chlorbenzaldehyd
m-Fluorbenzaldehyd
o-Trifluormethylbenzaldehyd
m-Trifluormethylbenzaldehyd
o-Methylbenzaldehyd
m-Methylbenzaldehyd
o-Methoxybenzaldehyd
m-Methoxybenzaldehyd
o-Trifluoräthoxybenzaldehyd
m-Trifluoräthoxybenzaldehyd
o-Methylmercaptobenzaldehyd
m-Methylmercaptobenzaldehyd
o-Cyclopropylbenzaldehyd
o-Azidobenzaldehyd, β-Azidobenzaldehyd
α-Pyridylaldehyd
β-Pyridylaldehyd

Als Verdünnungsmittel kommen Wasser und alle inerten organischen Lösungsmittel in Frage. Hierzu gehören vorzugsweise Alkohole wie Äthanol, Methanol, Isopropanol, Äther wie Dioxan, Diäthyläther, Tetrahydrofuran, Glykolmonomethyläther, Glykoldimethyläther oder Eisessig, Dimethylformamid, Dimethylsulfoxid, Acetonitril-und Pyridin.

Die Reaktionstemperaturen können in einem größeren Bereich variiert werden. Im allgemeinen arbeitet man zwischen 20 und 150°C, vorzugsweise bei der Siedetemperatur des jeweiligen Lösungsmittels.

Die Umsetzung kann bei Normaldruck, aber auch bei erhöhtem Druck durchgeführt werden. lm allgemeinen arbeitet man unter Normaldruck.

Bei der Durchführung des erfindungsgemäßen Verfahrens werden die an der Reaktion beteiligten Stoffe der Formeln II, III und IV vorzugsweise etwa in molaren Mengen eingesetzt.

Von besonderem Intessse sind fluorhaltige 1,4-Dihydropyridine der allgemeinen Formel in-welcher

### R für Wasserstoff steht,

R₁ und R⁶ gleich oder verschieden sind und für Alkyl mit 1 bis 4 Kahlenstoffatomen stehen,
R⁴ für Phenyl steht, welches gegebenenfalls durch ein oder zwei Substituenten aus der Gruppe Nitro, Cyano, Trifluormethyl, Fluor Chlor, Trifluormethoxy, Alkyl, Alkoxy oder Alkylmerkapto mit jeweils 1 bis 2 Kohlenstoffatomen in den Alkyl- und Alkoxyresten substituiert ist, oder für einen Pyridylrest steht,
R⁵ für Alkyl mit 1 bis 6 Kohlenstoffatomen steht, welches gegetienenfalls durch Fluor oder Chlor substituiert ist, oder für Alkoxyalkyl mit bis zu 6 Kohlenstoffatomen steht, oder für Aminoalkyl mit 1 bis 4 Kohlenstoffatomen im Alkylrest steht; wobei die beiden Wasserstoffatome der Aminogruppe durch 1 oder 2 Alkylgruppen mit 1 bis 4 Kohlenstoffatomen und/oder durch eine Benzylgruppe ersetzt sein können, oder für den Rest 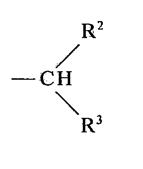 steht, wobei
R² und R³ jeweils gleich oder verschieden sind und wobei einer der beiden Substituenten Wasserstoff bedeuten kann und der andere Substituent oder beide Substituenten R² und R³ jeweils für einen geradkettigen verzweigten oder cyclischen Alkylrest mit bis zu 6 Kohlenstoffatomen stehen, der ein oder zwei perfluorierte Kohlenstoffatöme mit jeweils 2 oder 3 Fluosubstituenten enthält, wobei dieser Alkylrest gegebenenfalls durch die Gruppierung ―CH₂―O―CM₂― unterbrochen ist.

Außer den unten angeführten Herstellungsbeispielen seien noch folgende erfindungsgemäße Verbindungen hervorgehoben:

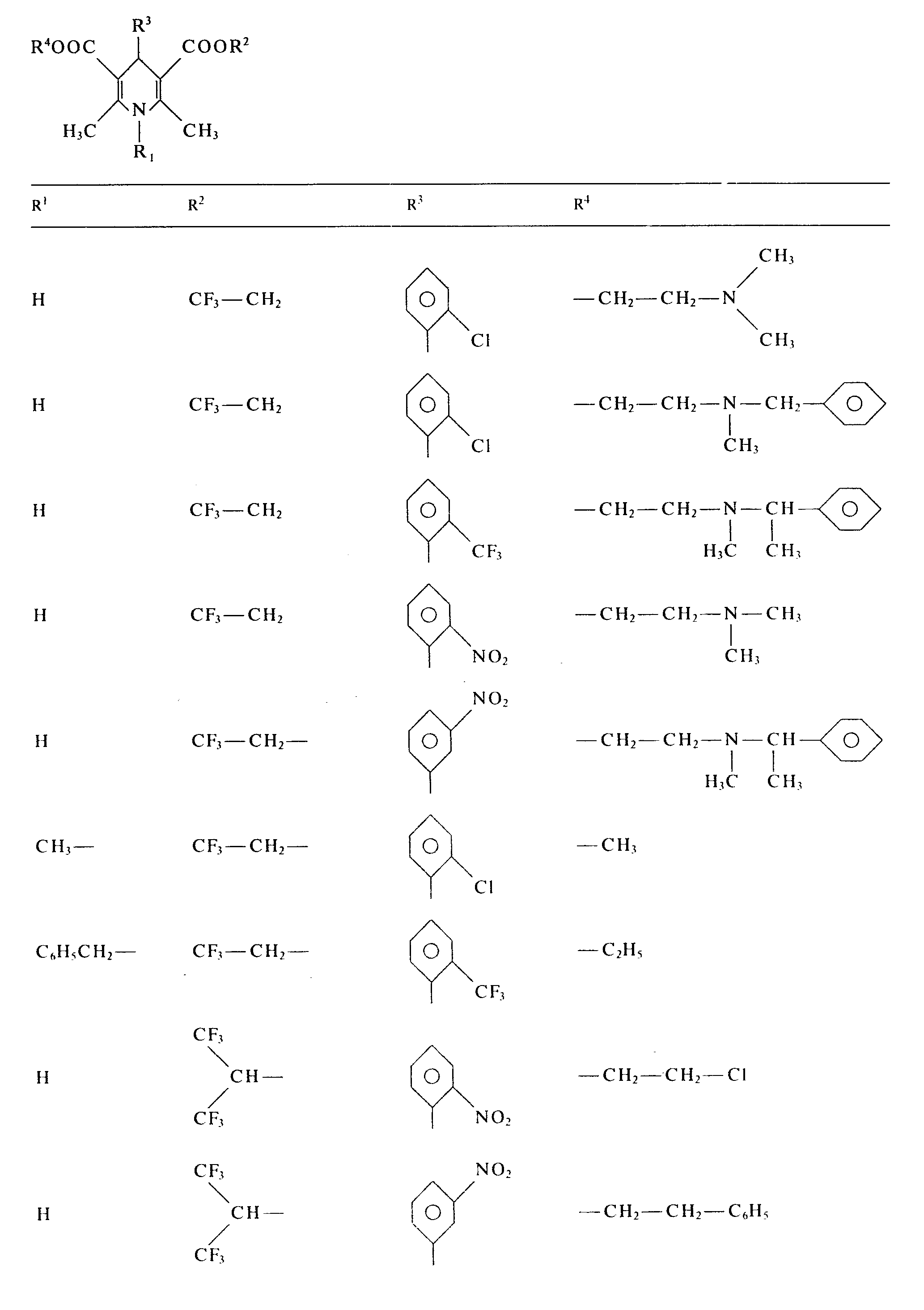

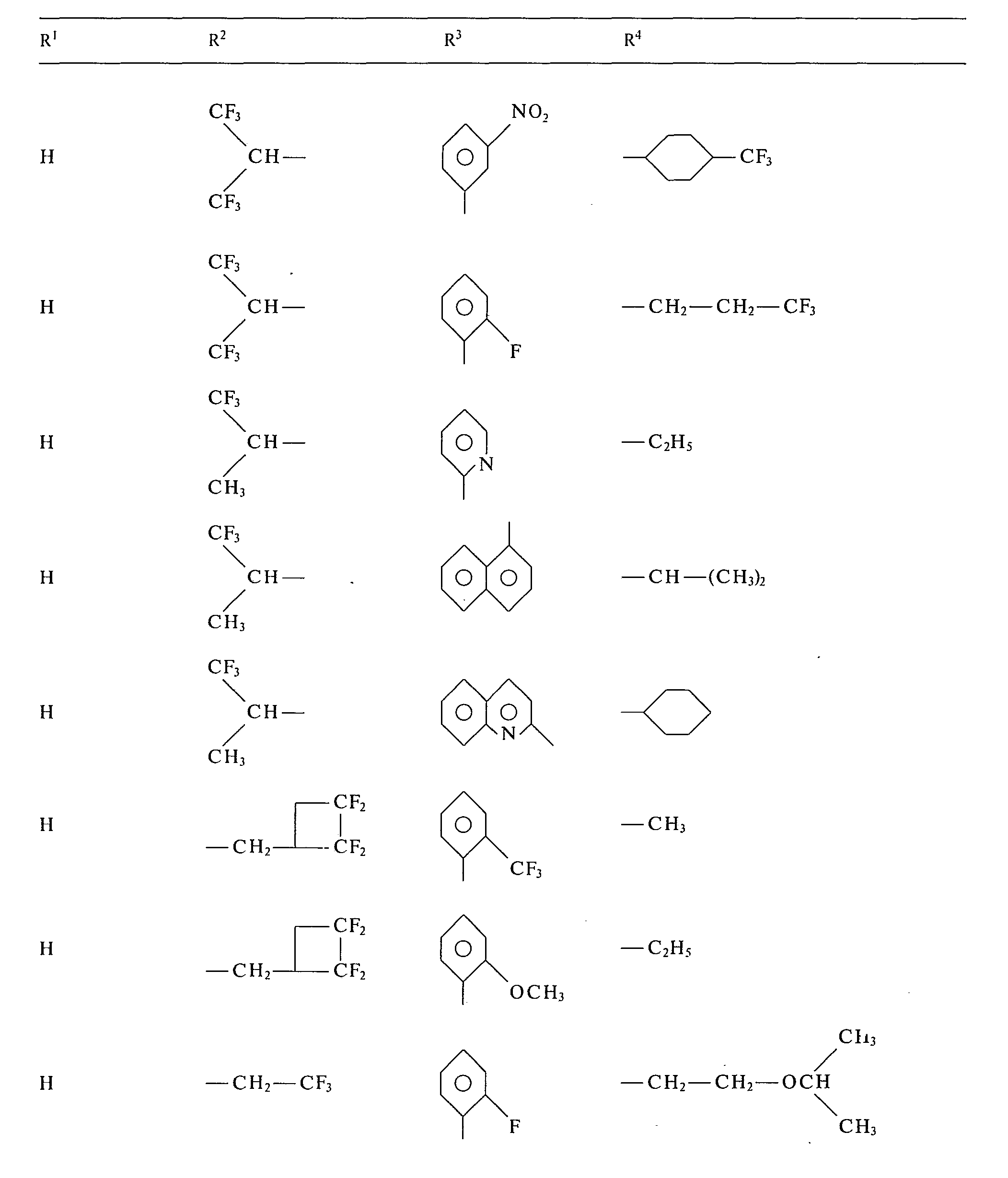

Die neuen Verbindungen sind als Arzneimittel verwendbare Substanzen. Sie haben ein breites und vielseitiges pharmakologisches Wirkungsspektrum. Im einzelnen konnten im Tierexperiment folgende Hauptwirkungen nachgewiesen werden:
1. Die Verbindungen bewirken bei parenteraler, oraler und perlingualer Zugabe eine deutliche und langanhaltende Erweiterung der Coronargefäße.
   Diese Wirkung auf die Coronargefäße wird durch einen gleichzeitigen nitritähnlichen, herzentlastenden Effekt verstärkt.
   Sie beeinflussen bzw. verändern den Herzstoffwechsel im Sinne einer Energieersparnis.
2. Die Erregbarkeit des Reizbildungs- und Erregungsleitungssystems innerhalb des Herzens wird herabgesetzt, so daß eine in therapeutischen Dosen nachweisbare Antiflimmerwirkung resultiert.
3. Der Tonus der glatten Muskulatur der Gefäße wird unter der Wirkung der Verbindungen stark vermindert. Diese gefäßspasmolytische Wirkung kann im gesamten Gefäßsystem stattfinden, oder sich mehr oder weniger isoliert in umschriebenen Gefäßgebieten (wie z. B. dem Zentralnervensystem) manifestieren.
4. Die Verbindungen senken den Blutdruck von normotonen und hypertonen Tieren und können somit als antihypertensive Mittel verwendet werden.
5. Die Verbindungen haben stark muskulär-spasmolytische Wirkung, die an der glatten Muskulatur des Magens, Darmtraktes, des Urogenitaltraktes und des Respirationssystems deutlich werden.

Die neuen Wirkstoffe können in bekannter Weise in die üblichen Formulierungen übergeführt werden, wie Tabletten, Kapseln, Dragees, Pillen, Granulate, Aerosole, Sirupe, Emulsionen, Suspensionen und Lösungen, unter Verwendung inerter, nichttoxischer, pharmazeutisch geeigneter Trägerstoffe oder Lösungsmittel. Hierbei soll die therapeutisch wirksame Verbindung jeweils in einer Konzentration von etwa 0,5 bis 90 Gew.-% der Gesamtmischung vorhanden sein, d. h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z. B. im Fall der Benutzung von Wasser als Verdünnungsmittel gegebenenfalls organische Lösungsmittel als Hilfslösungsmittel verwendet werden können.

Als Hilfsstoffe seien beispielhaft aufgeführt:
Wasser, nichttoxische organische Lösungsmittel, wie Paraffine (z. B. Erdölfraktionen), pflanzliche Öle (z. B. Erdnuß-/Sesamöl), Alkohole (z. B. Äthylalkohol, Glycerin), Glykole (z. B. Propylenglykol, Polyäthylenglykol), feste Trägerstoffe, wie z. B. natürliche Gesteinsmehle (z. B. Kaoline, Tonerden, Talkum, Kreide), synthetische Gesteinsmehle (z. B. hochdisperse Kieselsäure, Silikate), Zucker (z. B. Roh-, Milch- und Traubenzucker), Emulgiermittel, wie nichtionogene und anionische Emulgatoren (z. B. Polyoxyäthylen-Fettsäure-Ester, Polyoxyäthylen-Fett-alkohol-Äther, Alkylsulfonate und Arylsulfonate), Dispergiermittel (z. B. Lignin, Sulfitablaugen, Methylcellulose, Stärke und Polyvinylpyrrolidon) und Gleitmittel (z. B. Magnesiumstearat, Talkum, Stearinsäure und Natriumlaurylsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen und/oder Elixieren, die für orale Anwendungen gedacht sind, können die Wirkstoffe außer mit den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt-werden.

Im allgemeinen hat es sich als vorteilhaft erwiesen, bei intravenöser Applikation Mengen von etwa 0,001 bis 1 mg/kg, vorzugsweise etwa 0,005 bis 0,1 mg/kg Körpergewicht pro Tag zur Erzielung wirksamer Ergebnisse zu verabreichen, und bei oraler Applikation beträgt die Dosierung etwa 0,005 bis 10 mg/kg, vorzugsweise 0,05 bis 5 mg/kg Körpergewicht pro Tag.

Trotzdem kann es gegebenenfalls erforderlich sein, von den genannten Mengen abzuweichen, und zwar in Abhängigkeit vom Körpergewicht des Versuchstieres bzw. der Art des Applikationsweges, aber auch aufgrund der Tierart und deren individuellem Verhalten gegenüber dem Medikament bzw. der Art von dessen Formulierung und dem Zeitpunkt bzw. Intervall zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehrere Einzelgaben über den Tag zu verteilen. Für die Applikation in der Humanmedizin ist der gleiche Dosierungsspielraum vorgesehen. Sinngemäß gelten hierbei auch die obigen Ausführungen.

### Herstellungsbeispiele

### Beispiel 1

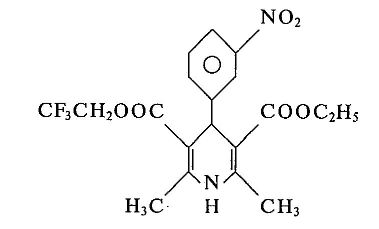

3-Äthoxycarbonyl-2,6-dimethyl-4-(3-nitrophenyl)-5-trifluoräthoxycarbonyl-1,4-dihydropyridin.

5 g Aminocrotonsäuretrifluoräthylester werden in 500 ml Ethanol mit 7,3 g m-Nitrobenzylidenacetessigsäureäthylester 3 Stunden unter Rückfluß erhitzt. Man kühlt, saugt ab und erhält 7,8 g schwach gelbe Kristalle vom Fp.: 192° Centsprechend 67% der Theorie.

### Beispiel 2

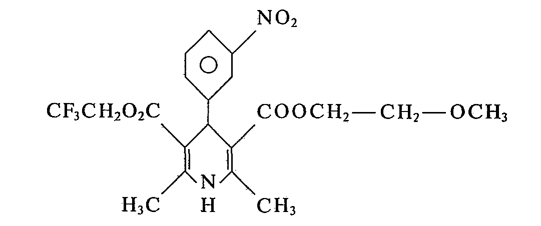

2,6-Dimethyl-3-ß-methoxyäthoxycarbonyl-4-(3-nitrophenyl)-5-trifluoräthoxycarbonyl-1,4-dihydropyridin.

5 g Aminocrotonsäuretrifluoräthylester werden zusammen mit 3,1 g m-Nitrobenzylidenacetessigsäure-ß-methoxyäthylester in 500 ml Propanol 3 Stunden unter Rückfluß erhitzt. Man saugt ab und erhält 9,3 g schwach gelbe Kristalle vom Fp.: 169° Centsprechend 74% der Theorie.

In analoger Arbeitsweise zu Beispiel 1 wurden die in der Tabelle aufgeführten Verbindungen erhalten.

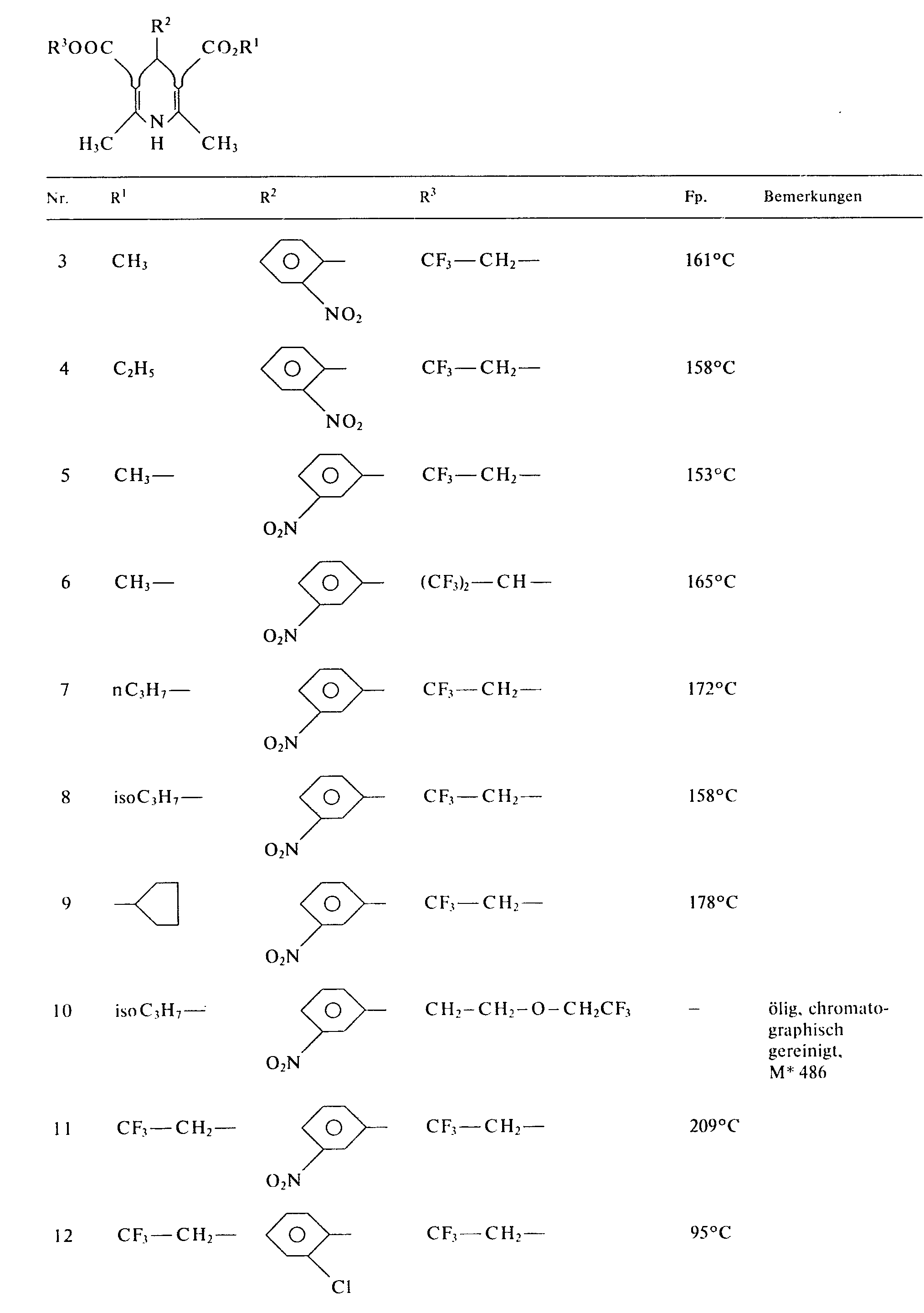

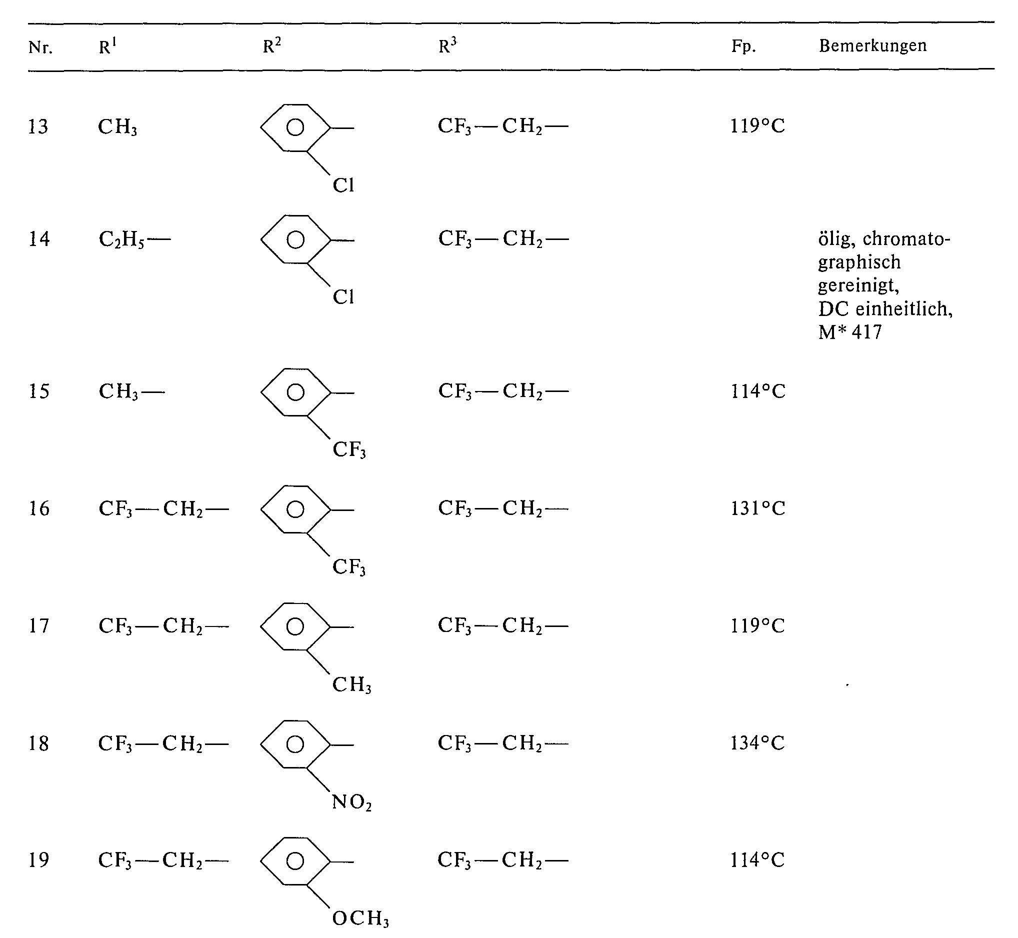

## Patentansprüche (Patentansprüche für folgende(n) Vertragsstaat(en) : BE, CH, DE, FR, GB, IT, NL, SE)

1. Fluorhaltige 1,4-Dihydropyridine der allgemeinen Formel 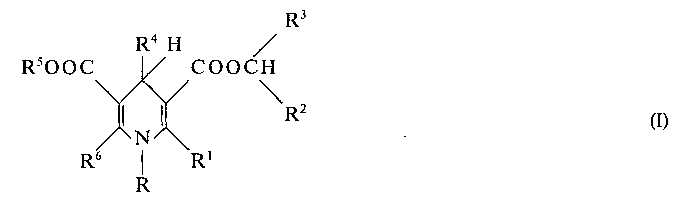 in welcher
R für Wasserstoff, Alkyl mit 1 bis 2 Kohlenstoffatomen oder Benzyl steht,
R¹ und R⁶ gleich oder verschieden sind und fürAlkyl mit 1 bis4 Kohlenstoffatomen stehen,
R⁴ für Phenyl steht, welches gegebenenfalls durch ein oder zwei Substituenten aus der Gruppe Nitro, Cyano, Trifluormethyl, Halogen, Acido, Trifluormethoxy, Alkyl, Alkoxy, Alkylmercapto oder S0₂-Alkyl mit jeweils 1 bis 2 Kohlenstoffatomen in den Alkyl- und Alkoxyresten substituiert ist oder für einen Pyridylrest steht,
R⁵ für Alkyl mit 1 bis 6 Kohlenstoffatomen, welches gegebenenfalls durch Halogen substituiert ist steht oder für Alkoxyalkyl, Alkenyl, Alkinyl mit bis zu 6 Kohlenstoffatomen im Alkylrest steht, wobei die beiden Wasserstoffatome der Aminogruppe durch 1 oder 2 Alkylgruppen mit 1 bis 4 Kohlenstoffatomen und/oder durch eine Benzylgruppe ersetzt sein können oder für den Rest 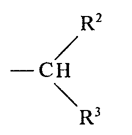 steht, und
R² und R³ jeweils gleich oder verschieden sind, wobei einer der beiden Substituenten Wasserstoff bedeuten kann und der andere Substituent oder beide Substituenten R² und R³ jeweils für einen geradkettigen, verzweigten oder cyclischen Alkylrest mit bis zu 8 Kohlenstoffatomen, vorzugsweise mit bis zu 6 Kohlenstoffatomen, stehen, der 1, 2 oder 3 perfluorierte Kohlenstoffatome mit jeweils 2 oder 3 Fluorsubstituenten enthält, wobei dieser Alkylrest gegebenenfalls durch die Gruppierung -CH₂-X-CH₂- unterbrochen ist, wobei X Sauerstoff oder Schwefel bedeutet oder wobei R² und R³ gemeinsam mit der CH-Gruppe einen gesättigten isocyclischen Rest mit 4 bis 7 Ringgliedern bilden können, der 1, oder 3 perfluorierte C-Atome enthält und der gegebenenfalls durch eine Alkylgruppe mit 1 bis 4 Kohlenstoffatomen substituiert ist, wobei diese Alkylgruppe ihrerseits wieder ein oder zwei perfluorierte Kohlenstoffatome enthalten kann, sowie ihre pharmazeutische unbedenklichen Säureadditionssalze.

2. Verbindungen der allgemeinen Formel 1 gemäß Anspruch 1, in welcher
R für Wasserstoff steht,
R^{l} und R⁶ gleich oder verschieden sind und für Alkyl mit 1 bis 4 Kohlenstoffatomen stehen,
R⁴ für Phenyl steht, welches gegebenenfalls durch ein oder zwei Substituenten aus der Gruppe Nitro, Cyano, Trifluormethyl, Fluor, Chlor, Trifluormethoxy, Alkyl, Alkoxy oder Alkylmerkapto mit jeweils 1 bis 2 Kohlenstoffatomen in den Alkyl- und Alkoxyresten substituiert ist, oder für einen Pyridylrest steht,
R⁵ für Alkyl mit 1 bis 6 Kohlenstoffatomen steht, welches gegebenenfalls durch Fluor oder Chlor substituiert ist, oder für Alkoxyalkyl mit bis zu 6 Kohlenstoffatomen steht, oder für Aminoalkyl mit 1 bis 4 Kohlenstoffatomen im Alkylrest steht, wobei die beiden Wasserstoffatome der Aminogruppe durch 1 oder 2 Alkylgruppen mit 1 bis 4 Kohlenstoffatomen und/oder durch eine Benzylgruppe ersetzt sein können, oder für den Rest 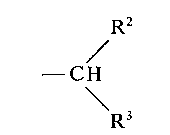 steht, wobei
R² und R³ jeweils gleich oder verschieden sind und wobei einer der beiden Substituenten Wasserstoff bedeuten kann und der andere Substituent oder beide Substituenten R² und R³ jeweils für einen geradkettigen, verzweigten oder cyclischen Alkylrest mit bis zu 6 Kohlenstoffatomen stehen, der ein oder zwei perfluorierte Kohlenstoffatome mit jeweils 2 oder 3 Fluorsubstituenten enthält, wobei dieser Alkylrest gegebenenfalls durch die Gruppierung -CH₂-0-CH₂- unterbrochen ist.

3. Verfahren zur Herstellung von fluorhaltigen 1,4-Dihydropyridinen der allgemeinen Formel 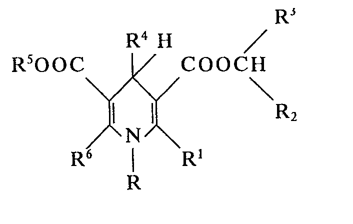 in welcher
R für Wasserstoff, Alkyl mit 1 bis 2 Kohlenstoffatomen oder Benzyl steht,
R^{l} und R⁶ gleich oder verschieden sind und für Alkyl mit 1 bis 4 Kohlenstoffatomen stehen,
R⁴ für Phenyl steht, welches gegebenenfalls durch ein oder zwei Substituenten aus der Gruppe Nitro, Cyano, Trifluormethyl, Halogen, Acido, Trifluormethoxy, Alkyl, Alkoxy, Alkylmercapto oder S0₂-Alkyl mit jeweils 1 bis 2 Kohlenstoffatomen in den Alkyl- und Alkoxyresten substituiert ist oder für einen Pyridylrest steht,
R⁵ für Alkyl mit 1 bis 6 Kohlenstoffatomen, welches gegebenenfalls durch Halogen substituiert ist, steht, oder für Alkoxyalkyl, Alkenyl, Alkinyl mit bis zu 6 Kohlenstoffatomen steht oder für Aminoalkyl mit 1 bis 4 Kohlenstoffatomen im Alkylrest steht, wobei die beiden Wasserstoffatome der Aminogruppe durch 1 oder 2 Alkylgruppen mit 1 bis 4 Kohlenstoffatomen und/oder durch eine Benzylgruppe ersetzt sein können oder für den Rest 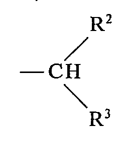 und
R² und R³ jeweils gleich oder verschieden sind, wobei einer der beiden Substituenten Wasserstoff bedeuten kann und der andere Substituent oder beide Substituenten R² und R³ jeweils für einen geradkettigen, verzweigten oder cyclischen Alkylrest mit bis zu 8 Kohlenstoffatomen, vorzugsweise mit bis zu 6 Kohlenstoffatomen stehen, der 1, 2 oder 3 perfluorierte Kohlenstoffatome mit jeweils 2 oder 3 Fluorsubstituenten enthält, wobei dieser Alkylrest gegebenenfalls durch die Gruppierung -CH₂-X-CH₂- unterbrochen ist, wobei X Sauerstoff oder Schwefel bedeutet, oder wobei
R² und R³ gemeinsam mit der CH-Gruppe einen gesättigten isocyclischen Rest mit 4 bis 7 Ringgliedern bilden können, der 1, 2 oder 3 perfluorierte C-Atome enthält und der gegebenenfalls durch eine Alkylgruppe mit 1 bis 4 Kohlenstoffatomen substituiert ist, wobei diese Alkylgruppe ihrerseits wieder ein oder zwei perfluorierte Kohlenstoffatome enthalten kann, sowie ihre pharmazeutisch unbedenklichen Säureadditionssalze,
dadurch gekennzeichnet, daß man 1 Mol Enamin der allgemeinen Formel il 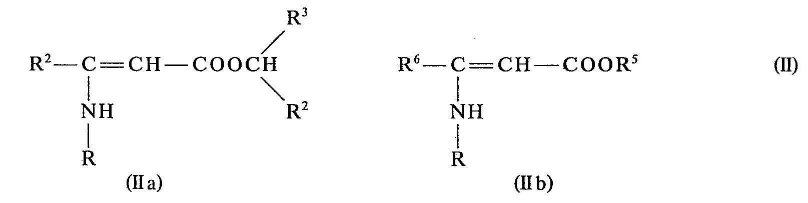 in welcher
R, Rl, R2, R³, R⁵ und R⁶ die oben angegebene Bedeutung haben,
mit einem Mol β-Ketocarbonsäureester der allgemeinen Formel III 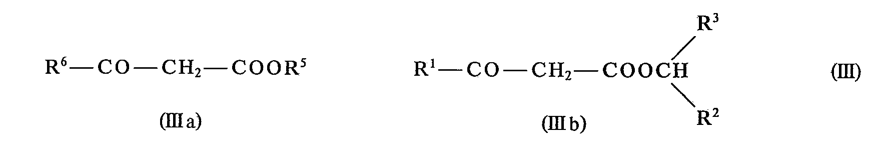 in welcher
Rl, R2, R³, R⁵ und R⁶ die oben angegebene Bedeutung haben, mit einem Mol Aldehyd der allgemeinen Formel (IV) 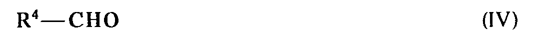 in welcher
R⁴ die oben angegebene Bedeutung besitzt,
gegebenenfalls nach Isolierung der sich aus (III) und (IV) bildenden Ylideiwerbindungen der allgemeinen Formel (V) 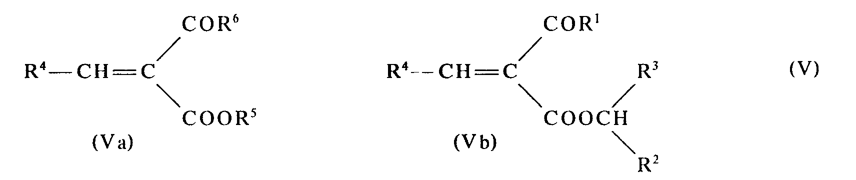 in welcher
R', R2, R3, R⁴, R⁵ und R⁶ die oben angegebene Bedeutung besitzen,
gegebenenfalls in Gegenwart von Wasser oder inerter organischer Lösungsmittel bei Temperaturen zwischen 20 und 150° C umsetzt.

4. Arzneimittel enthaltend mindestens ein fluorhaltiges 1,4-Dihydropyridin der allgemeinen Formel gemäß Anspruch 1.

5. Verfahren zur Herstellung von Arzneimitteln, dadurch gekennzeichnet, daß man Verbindungen der allgemeinen Formel gemäß Anspruch 1 bzw. Anspruch 3 gegebenenfalls unter Verwendung von inerten Hilfs- und Trägerstoffen in eine geeignete Applikationsform überführt.

## Patentansprüche (Patentansprüche für folgende(n) Vertragsstaat(en) : AT)

1. Verfahren zur Herstellung von fluorhaltigen 1,4-Dihydropyridinen der allgemeinen Formel 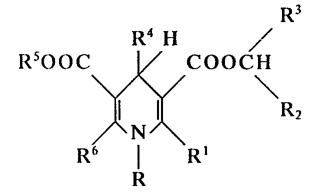 in welcher
R für Wasserstoff, Alkyl mit 1 bis 2 Kohlenstoffatomen oder Benzyl steht,
R' und R⁶ gleich oder verschieden sind und für Alkyl mit 1 bis 4 Kohlenstoffatomen stehen,
R⁴ für Phenyl steht, welches gegebenenfalls durch ein oder zwei Substituenten aus der Gruppe Nitro, Cyano, Trifluormethyl, Halogen, Acido, Trifluormethoxy, Alkyl, Alkoxy, Alkylmercapto oder S0₂-Alkyl mit jeweils 1 bis 2 Kohlenstoffatomen in den Alkyl- und Alkoxyresten substituiert ist oder für einen Pyridylrest steht,
R⁵ für Alkyl mit 1 bis 6 Kohlenstoffatomen, welches gegebenenfalls durch Halogen substituiert ist, steht, oder für Alkoxyalkyl, Alkenyl, Alinyl mit bis zu 6 Kohlenstoffatomen steht oder für Aminoalkyl mit 1 bis 4 Kohlenstoffatomen im Alkylrest steht, wobei die beiden Wasserstoffatome der Aminogruppe durch 1 oder 2 Alkylgruppen mit 1 bis 4 Kohlenstoffatomen und/oder durch eine Benzylgruppe ersetzt sein können oder für den Rest 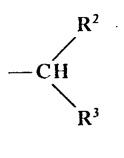 steht, und
R² und R³ jeweils gleich oder verschieden sind, wobei einer der beiden Substituenten Wasserstoff bedeuten kann und der andere Substituent oder beide Substituenten R² und R³ jeweils für einen geradkettigen, verzweigten oder cyclischen Alkylrest mit bis zu 8 Kohlenstoffatomen, vorzugsweise mit bis zu 6 Kohlenstoffatomen stehen, der 1, 2 oder 3 perfluorierte Kohlenstoffatome mit jeweils 2 oder 3 Fluorsubstituenten enthält, wobei dieser Alkylrest gegebenenfalls durch die Gruppierung -CH₂-X-CH₂- unterbrochen ist, wobei X Sauerstoff oder Schwefel bedeutet, oder wobei
R² und R³ gemeinsam mit der CH-Gruppe einen gesättigten isocyclischen Rest mit 4 bis 7 Ringgliedern bilden können, der 1, 2 oder 3 perfluorierte C-Atome enthält und der gegebenenfalls durch eine Alkylgruppe mit 1 bis 4 Kohlenstoffatomen substituiert ist, wobei diese Alkylgruppe ihrerseits wieder ein oder zwei perfluorierte Kohlenstoffatome enthalten kann, sowie ihre pharmazeutisch unbedenklichen Säureadditionssalze,
dadurch gekennzeichnet, daß man 1 Mol Enamin der allgemeinen Formel II 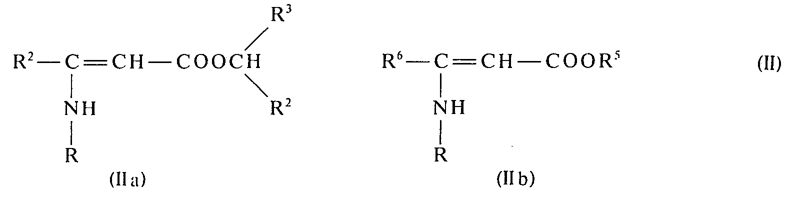 in welcher
R, R^{I}, R², R³, R⁵ und R⁶ die oben angegebene Bedeutung haben,
mit einem Mol β-Ketocarbonsäureester der allgemeinen Formel III 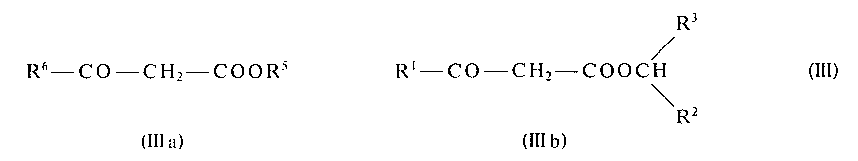 in welcher
R¹, R², R³, R⁵ und R⁶ die oben angegebene Bedeutung haben,
mit einem Mol Aldehyd der allgemeinen Formel (IV) 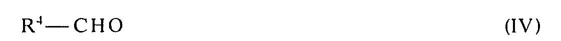 in welcher
R⁴ die oben angegebene Bedeutung besitzt,
gegebenenfalls nach Isolierung der sich aus (III) und (IV) bildenden Ylidenverbindungen der allgemeinen Formel (V) 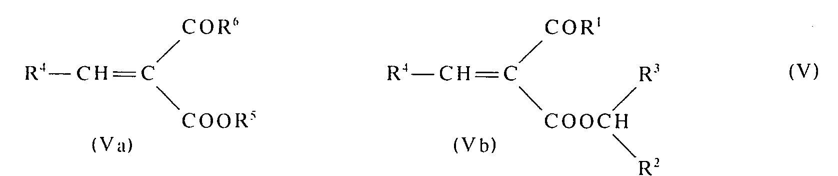 in welcher
R', R2, R³, R⁴, R⁵ und R⁶ die oben angegebene Bedeutung besitzen, gegebenenfalls in Gegenwart von Wasser oder inerter organischer Lösungsmittel bei Temperaturen zwischen 20 und 150° C umsetzt.

## Claims (Claims for the following Contracting State(s) : s: BE, CH, DE, FR, GB, IT, NI, SE)

1. Fluorine-containing 1,4-dihydropyridines of the general formula I 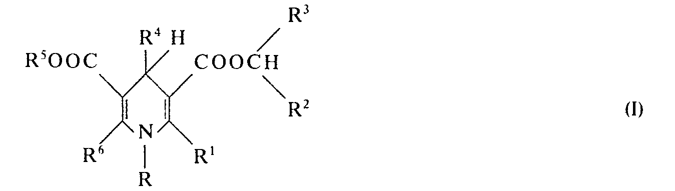
R represents hydrogen, alkyl with 1 to 2 carbon atoms or benzyl,
R' and R⁶ are identical or different and represent alkyl with 1 to 4 carbon atoms,
R⁴ represents phenyl, which is optionally substituted by one or two substituents from the group comprising nitro, cyano, trifluoromethyl, halogen, acido, trifluoromethoxy, alkyl, alkoxy, alkylmercapto or S0₂-alkyl, in each case with 1 to 2 carbon atoms in the alkyl and alkoxy radicals, or represents a pyridyl radical,
R⁵ represents alkyl with 1 to 6 carbon atoms, which is optionally substituted by halogen, or represents alkoxyalkyl, alkenyl or alkynyl with up to 6 carbon atoms in the alkyl radical, ist being possible for the two hydrogen atoms of the amino group to be replaced by 1 or 2 alkyl groups with 1 to 4 carbon atoms and/or by a benzyl group, or represents the radical 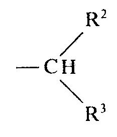 and
R² and R³ in each case are identical or different and one of the two substituents can denote hydrogen and the other substituent, or the two substituents R² and R³, in each case represents or represent a straight-chain, branched or cyclic alkyl radical which has up to 8 carbon atoms and preferably up to 6 carbon atoms and contains 1, 2 or 3 perfluorinated carbon atoms with in each case, 2 or 3 fluorine substituents, this alkyl radical being optionally interrupted by the grouping -CH₂-X-CH₂-, in which X denotes oxygen or sulphur, or in which
R² and R³ together with the CH group can form a saturated isocyclic radical which has 4 to 7 ring members and contains 1, 2 or 3 perfluorinated C atoms and is optionally substituted by an alkyl group with 1 to 4 carbon atoms, it being possible for this alkyl group, in turn, again to contain one or two perfluorinated carbon atoms, and their pharmaceutically acceptable acid addition salts.

2. Compounds of the general formula I according to Claim 1 in which
R represents hydrogen,
R' and R⁶ are identical or different and represent alkyl with 1 to 4 carbon atoms,
R⁴ represents phenyl, which is optionally substituted by one or two substituents from the group comprising nitro, cyano, trifluoromethyl, fluorine, chlorine, trifluoromethoxy, alkyl, alkoxy or alkylmercapto in each case with 1 to 2 carbon atoms in the alkyl and alkoxy radicals, or represents a pyridyl radical,
R⁵ represents alkyl with 1 to 6 carbon atoms, which is optionally substituted by fluorine or chlorine, or represents alkoxyalkyl with up to 6 carbon atoms or represents aminoalkyl with 1 to 4 carbon atoms in the alkyl radical, it being possible for the two hydrogen atoms of the amino group to be replaced by 1 or 2 alkyl groups with 1 to 4 carbon atoms and/or by a benzyl group, or represents the radical 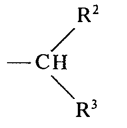 and
R² and R³ in each case are identical or different and one of the two substituents can denote hydrogen and the other substituent, or the two substituents R² and R³, in each case represents or represent a straight-chain, branched or cyclic alkyl radical which has up to 6 carbon atoms and contains one or two perfluorinated carbon atoms with in each case, 2 or 3 fluorine substituents, this alkyl radical being optionally interrupted by the grouping -CH₂-0-CH₂-.

3. Process for the preparation of fluorine-containing 1,4-dihydropyridines of the general formula I 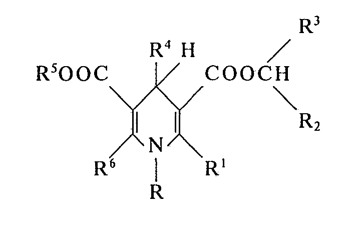 in which
R represents hydrogen, alkyl with 1 to 2 carbon atoms or benzyl,
R¹ and R⁶ are identical or different and represent alkyl with 1 to 4 carbon atoms,
R⁴ represents phenyl, which is optionally substituted by one or two substituents from the group comprising nitro, cyano, trifluoromethyl, halogen, acido, trifluoromethoxy, alkyl, alkoxy, alkylmercapto or S0₂-alkyl, in each case with to 2 carbon atoms in the alkyl and alkoxy radicals, or represents a pyridyl radical,
R⁵ represents alkyl with 1 to 6 carbon atoms, which is optionally substituted by halogen, or represents alkoxyalkyl, alkenyl or alkynyl with up to 6 carbon atoms or represents aminoalkyl with1 to 4 carbon atoms in the alkyl radical, it being possible for the two hydrogen atoms of the amino group to be replaced by 1 or 2 alkyl groups with 1 to 4 carbon atoms and/or by a benzyl group, or represents the radical 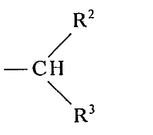 and
R² and R³ in each case are identical or different and one of the two substituents can denote hydrogen and the other substituent, or the two substituents R² and R³, in each case represents or represent a straight-chain, branched or cyclic alkyl radical which has up to 8 carbon atoms and preferably up to 6 carbon atoms and contains 1, 2 or 3 perfluorinated carbon atoms with in each case, 2 or 3 fluorine substituents, this alkyl radical being optionally interrupted by the grouping ―CH₂―X―CH₂―, in which X denotes oxygen or sulphur, or in which
R² and R³ together with the CH group can form a saturated isocyclic radical which has 4 to 7 ring members and contains 1, 2 or 3 perfluorinated C atoms and is optionally substituted by an alkyl group with 1 to 4 carbon atoms, it being possible for this alkyl group, in turn, again to contain one or two perfluorinated carbon atoms, and their pharmaceutically acceptable salts,
characterised in that 1 mol of an enamine of the general formula II 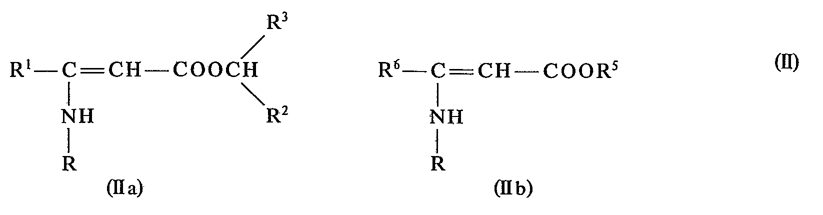 in which
R, R¹, R², R³, R⁵ and R⁶ have the meaning indicated above,
is reacted with one mol of a (β-ketocarboxylic acid ester of the general formula III 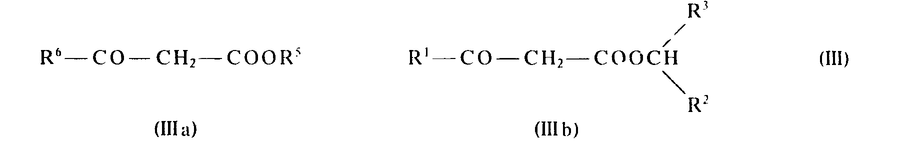 in which
R', R², R³, R⁵ and R⁶ have the meaning indicated above,
and with one mol of an aldehyde of the general formula (IV) 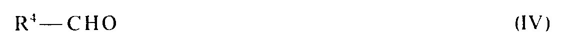 in which
R⁴ has the meaning indicated above,
optionally after isolation of the ylidene compounds which form from (III) and (IV) and have the general formula (V) 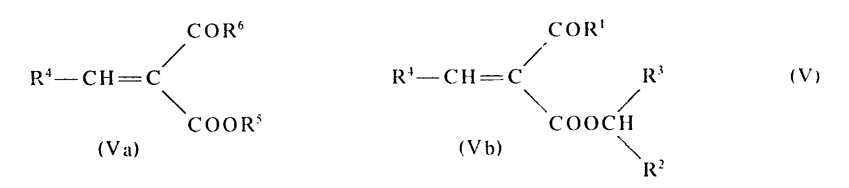 in which
R', R², R³, R⁴, R⁵ and R^{s} have the meaning indicated above,
optionally in the presence of water or inert organic solvents at temperatures between 20 and 150 C.

4. Medicament containing at least one fluorine-containing 1,4-dihydropyridine of the general formula I according to Claim 1.

5. Process for the preparation of medicaments, characterised in that compounds of the general formula I according to Claim 1 are converted, optionally with the use of inert auxiliary substances and excipients, to a suitable form for administration.

## Claims (Claims for the following Contracting State(s) : AT)

1. Process for the preparation of fluorine-containing 1,4-dihydropyridines of the general formula I 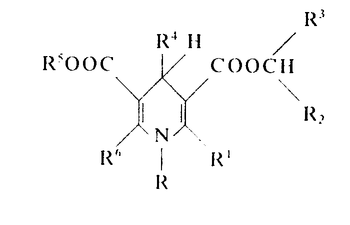 in which
R represents hydrogen, alkyl with 1 to 2 carbon atoms or benzyl,
R^{l} and R⁶ are identical or different and represent alkyl with 1 to 4 carbon atoms,
R⁴ represents phenyl, which is optionally substituted by one or two substituents from the group comprising nitro, cyano, trifluoromethyl, halogen, acido, trifluoromethoxy, alkyl, alkoxy, alkylmercapto or S0₂-alkyl, in each case with 1 to 2 carbon atoms in the alkyl and alkoxy radicals, or represents a pyridyl radical,
R⁵ represents alkyl with 1 to 6 carbon atoms, which is optionally substituted by halogen, or represents alkoxyalkyl, alkenyl or alkynyl with up to 6 carbon atoms or represents aminoalkyl with 1 to 4 carbon atoms in the alkyl radical, it being possible for the two hydrogen atoms of the amino group to be replaced by 1 or 2 alkyl groups with 1 to 4 carbon atoms and/or by a benzyl group, or represents the radical 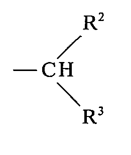 and
R² and R³ in each case are identical or different and one of the two substituents can denote hydrogen and the other substituent, or the two substituents R² and R³, in each case represents or represent a straight-chain, branched or cyclic alkyl radical which has up to 8 carbon atoms and preferably up to 6 carbon atoms and contains 1, 2 or 3 perfluorinated carbon atoms with in each case, 2 or 3 fluorine substituents, this alkyl radical being optionally interrupted by the grouping --CH₂-X-CH₂-, in which X denotes oxygen or sulphur, or in which
R² and R³ together with the CH group can form a saturated isocyclic radical which has 4 to 7 ring members and contains 1, 2 or 3 perfluorinated C atoms and ist optionally substituted by an alkyl group with 1 to 4 carbon atoms, it being possible for this alkyl group, in turn, again to contain one or two perfluorinated carbon atoms, and their pharmaceutically acceptable salts,
characterised in that 1 mol of an enamine of the general formula II 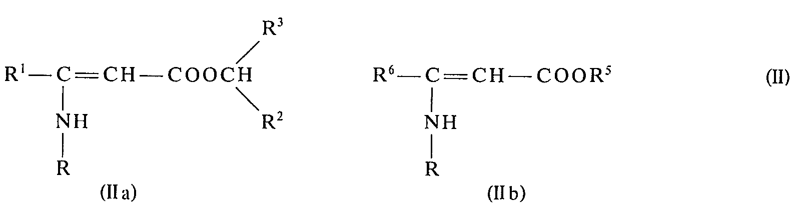 in which
R, R', R², R³, R⁵ and R⁶ have the meaning indicated above,
is reacted with one mol of a β-ketocarboxylic acid ester of the general formula III 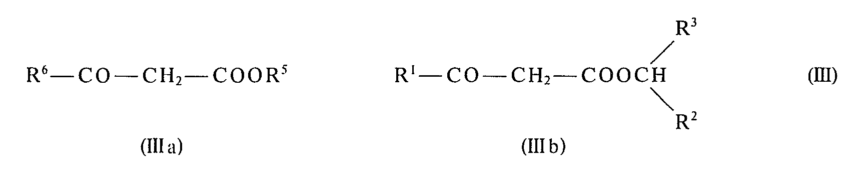 in which
R¹, R2, R3, R⁵ and R⁶ have the meaning indicated above, and with one mol of an aldehyde of the general formula (IV) 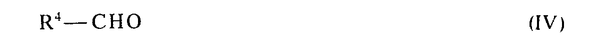 in which
R⁴ has the meaning indicated above,
optionally after isolation of the ylidene compounds which form from (III) and (IV) and have the general formula (V) 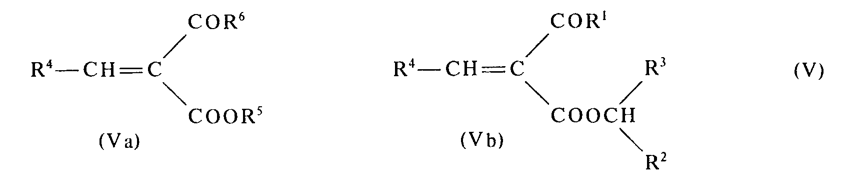 in which
R¹, R², R³, R⁴, R⁵ and R⁶ have the meaning indicated above,
optionally in the presence of water or inert organic solvents at temperatures between 20 and 150° C.

## Revendications (Revendications pour l'(les) Etat(s) contractant(s) suivant(s) : BE, CH, DE, FR, GB, IT, NL, SE)

1. 1,4-dihydropyridines contenant du fluor, de formule générale I: 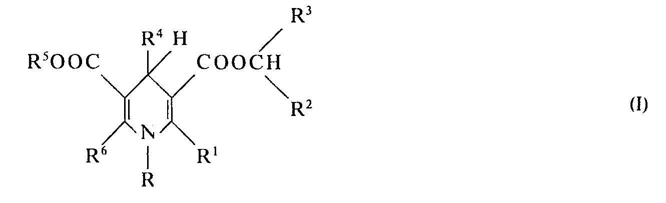 dans laquelle
R représente de l'hydrogène, un alcoyle ayant 1 à 2 atomes de carbone ou benzyle,
R¹ et R⁶ sont identiques ou différents et représentent un alcoyle ayant 1 à 4 atomes de carbone,
R⁴ représente un phényle qui est éventuellement substitué par un ou deux substituants appartenant au groupe nitro, cyano, trifluorométhyle, halogène, acido, trifluorométhoxy, alcoyle, alcoxy, alcoylmercapto ou S0₂-alcoyle ayant chaque fois 1 à 2 atomes de carbone dans les radicaux alcoyle et alcoxy, ou représente un radical pyridyle,
R⁵ représente un alcoyle ayant 1 à 6 atomes de carbone qui est éventuellement substitué par de l'halogène, ou représente un alcoxyalcoyle, alcényle, alcinyle ayant jusqu'à 6 atomes de carbone dans le radical alcoyle, les deux atomes d'hydrogène du groupe amino pouvant être remplacés par 1 ou 2 groupes alcoyle ayant 1 à 4 atomes de carbone et/ou par un groupe benzyle, ou représente le radical 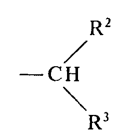 et
R² et R³ sont chaque fois identiques ou différents, un des deux substituants pouvant signifier de l'hydrogène et l'autre substituant, ou les deux substituants R² et R³, représentant chaque fois un radical alcoyle à chaîne droite, ramifiée ou cyclique ayant jusqu'à 8 atomes de carbone, de préférence jusqu'à 6 atomes de carbone, qui contient 1, ou 3 atomes de carbone perfluorés ayant chaque fois 2 ou 3 substituants fluor, ce radical alcoyle étant éventuellement interrompu par le groupement -CH₂-X-CH₂- où X signifie de l'oxygène ou du soufre, ou
R² et R³, ensemble avec le groupe CH, peuvent former un radical isocyclique saturé ayant 4 à 7 chaînons nucléaires qui contient 1, 2 ou 3 atomes de carbone perfluorés et qui est éventuellement substitué par un groupe alcoyle ayant 1 à 4 atomes de carbone, ce groupe alcoyle à son tour pouvant de nouveau contenir un ou deux atomes de carbone perfluorés, ainsi que leurs sels d'addition d'acides pharmaceutiquement acceptables.

2. Composés de formule générale 1 selon la revendication 1, dans laquelle
R représente de l'hydrogène,
R' et R⁶ sont identiques ou différents et représentent un alcoyle ayant 1 à 4 atomes de carbone,
R⁴ représente un phényle qui est substitué éventuellement par un ou deux substituants appartenant au groupe nitro, cyano, trifluorométhyle, fluor, chlore, trifluorométhoxy, alcoyle, alcoxy ou alcoylmercapto avec chaque fois 1 à 2 atomes de carbone dans les radicaux alcoyle et alcoxy, ou représente un radical pyridyle,
R⁵ représente un alcoyle ayant 1 à 6 atomes de carbone qui est éventuellement substitué par du fluor ou du chlore, ou représente un alcoxyalcoyle ayant jusqu'à 6 atomes de carbone, ou un aminoalcoyle ayant 1 à 4 atomes de carbone dans le radical alcoyle, les deux atomes d'hydrogène du groupe amino pouvant être remplacés par 1 ou 2 groupes alcoyle ayant 1 à 4 atomes de carbone et/ou par un groupe benzyle, ou représente le radical 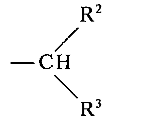 R² et R³ étant chaque fois identiques ou différents et un des deux substituants pouvant signifier de l'hydrogène et l'autre substituant, ou les deux substituants R² et R³, représentant chaque fois un radical alcoyle à chaîne droite, ramifiée ou cyclique ayant jusqu'à 6 atomes de carbone qui contient un ou deux atomes de carbone perfluorés ayant chacun 2 ou 3 substituants fluor, ce radical alcoyle étant éventuellement interrompu par le groupement -CH₂-0-CH₂-.

3. Procédé de préparation de 1,4-dihydropyridines contenant du fluor, de formule générale I: 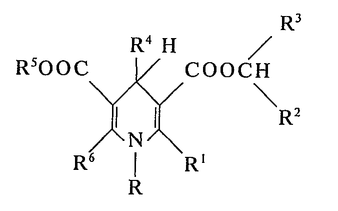 dans laquelle
R représente de l'hydrogène, alcoyle ayant 1 à 2 atomes de carbone ou benzyle,
R' et R⁶ sont identiques ou différents et représentent un alcoyle ayant 1 à 4 atomes de carbone,
R⁴ représente un phényle qui est substitué éventuellement par un ou deux substituants appartenant au groupe nitro, cyano, trifluorométhyle, halogène, acido, trifluorométhoxy, alcoyle, alcoxy, alcoylmercapto ou S0₂-alcoyle ayant chaque fois 1 à 2 atomes de carbone dans les radicaux alcoyle et alcoxy, ou représente un radical pyridyle,
R⁵ représente un alcoyle ayant 1 à 6 atomes de carbone qui est éventuellement substitué par de l'halogène, ou représente un alcoxyalcoyle, alcényle, alcinyle ayant jusqu'à 6 atomes de carbone, ou représente un aminoalcoyle ayant 1 à 4 atomes de carbone dans le radical alcoyle, les deux atomes d'hydrogène du groupe amino pouvant être remplacés par 1 ou 2 groupes alcoyle ayant 1 à 4 atomes de carbone et/ou par un groupe benzyle, ou représente le radical 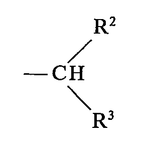 et
R² et R³ sont chaque fois identiques ou différents, un des deux substituants pouvant signifier de l'hydrogène et l'autre substituant, ou les deux substituants R² et R³, représentant chaque fois un radical alcoyle à chaîne droite, ramifiée ou cyclique ayant jusqu'à 8 atomes de carbone, de préférence jusqu'à 6 atomes de carbone, qui contient 1, ou 3 atomes de carbone perfluorés ayant chaque fois 2 ou 3 substituants fluor, ce radical alcoyle étant éventuellement interrompu par le groupement -CH₂-X-CH₂-, où X signifie de l'oxygène ou du soufre, ou R² et R³, en commun avec le groupe CH, peuvent former un radical isocyclique saturé ayant 4 à 7 chaînons nucléaires, qui contient 1, 2 ou 3 atomes de carbone perfluorés et qui est éventuellement substitué par un groupe alcoyle ayant 1 à 4 atomes de carbone, ce groupe alcoyle à son tour pouvant de nouveau contenir un ou deux atomes de carbone perfluorés, ainsi que de leurs sels pharmaceutiquement acceptables,
caractérisé en ce qu'on fait réagir 1 mole d'ènamine de formule générale Il 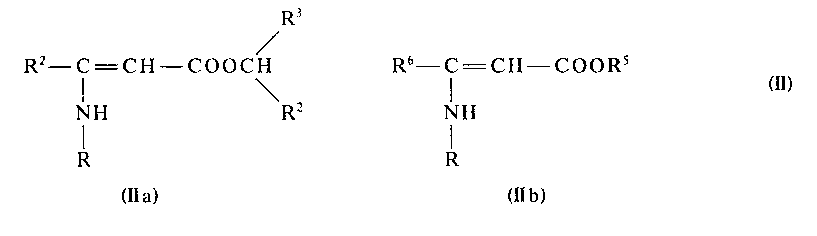 dans laquelle
R, R¹, R², R³, R⁵ et R⁶ ont la signification indiquée ci-dessus,
avec une mole d'ester d'acide β-cétocarboxylique de formule générale III 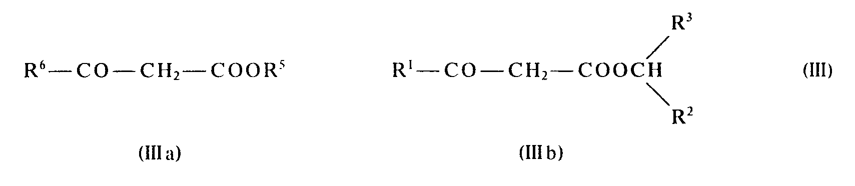 dans laquelle
R¹, R², R³, R⁵ et R⁶ ont la signification indiquée ci-dessus,
avec une mole d'aldéhyde de formule générale (IV) 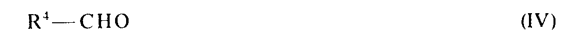 dans laquelle
R⁴ possède la signification indiquée ci-dessus,
éventuellement après isolation des composés ylidène se formant à partir de (III) et de (IV), de formule générale (V): 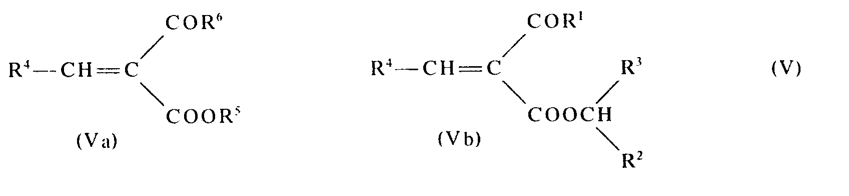
dans laquelle
R¹, R², R³, R⁴, R⁵ et R⁶ possèdent la signification indiquée ci-dessus,
éventuellement en présence d'eau ou de solvants organiques inertes à des températures entre 20 et 150⁰ _{C.}

4. Médicaments contenant au moins une 1,4-dihydropyridine contenant du fluor de formule générale 1 selon la revendication 1.

5. Procédé de fabrication de médicaments, caractérisé en ce qu'on convertit des composés de formule générale I selon la revendication 1, éventuellement avec utilisation de substance auxiliaires et de support inertes, en une forme d'application appropriée.

## Revendications (Revendications pour l'(les) Etat(s) contractant(s) suivant(s) : AT)

1. Procédé de préparation de 1,4-dihydropyridines contenant du fluor, de formule générale I: 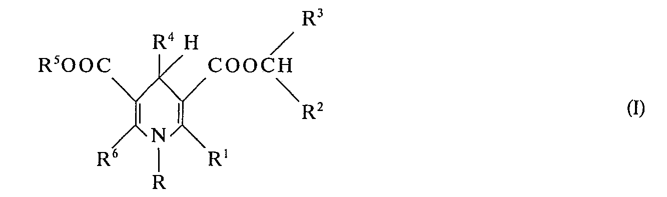 dans laquelle
R représente de l'hydrogène, alcoyle ayant 1 à 2 atomes de carbone ou benzyle,
R' et R⁶ sont identiques ou différents et représentant un alcoyle ayant 1 à 4 atomes de carbone,
R⁴ représente un phényle qui est substitué éventuellement par un ou deux substituants appartenant au groupe nitro, cyano, trifluorométhyle, halogène, acido, trifluorométhoxy, alcoyle, alcoxy, alcoylmercapto ou S0₂-alcoyle ayant chaque fois 1 à 2 atomes de carbone dans les radicaux alcoyle et alcoxy, ou représente un radical pyridyle,
R⁵ représente un alcoyle ayant 1 à 6 atomes de carbone qui est éventuellement substitué par de l'halogène, ou représente un alcoxyalcoyle, alcényle, alcinyle ayant jusqu'à 6 atomes de carbone, ou représente un aminoalcoyle ayant 1 à 4 atomes de carbone dans le radical alcoyle, les deux atomes d'hydrogène du groupe amino pouvant être remplacés par 1 ou 2 groupes alcoyle ayant 1 à 4 atomes de carbone et/ou par un groupe benzyle, ou représente le radical 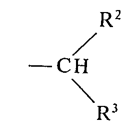 et
R² et R³ sont chaque fois identiques ou différents, un des deux substituants pouvant signifier de l'hydrogène et l'autre substituant, ou les deux substituants R² et R³, représentant chaque fois un radical alcoyle à chaîne droite, ramifiée ou cyclique ayant jusqu'à 8 atomes de carbone, de préférence jusqu'à 6 atomes de carbone, qui contient 1, ou 3 atomes de carbone perfluorés ayant chaque fois 2 ou 3 substituants fluor, ce radical alcoyle étant éventuellement interrompu par le groupement -CH₂-X-CH₂-, où X signifie de l'oxygène ou du soufre, ou R² et R³, en commun avec le groupe CH, peuvent former un radical isocyclique saturé ayant 4 à 7 chaînons nucléaires, qui contient 1, 2 ou 3 atomes de carbone perfluorés et qui est éventuellement substitué par un groupe alcoyle ayant 1 à 4 atomes de carbone, ce groupe alcoyle à son tour pouvant de nouveau contenir un ou deux atomes de carbone perfluorés, ainsi que de leurs sels pharmaceutiquement acceptables,
caractérisé en ce qu'on fait réagir 1 mole d'énamine de formule générale II 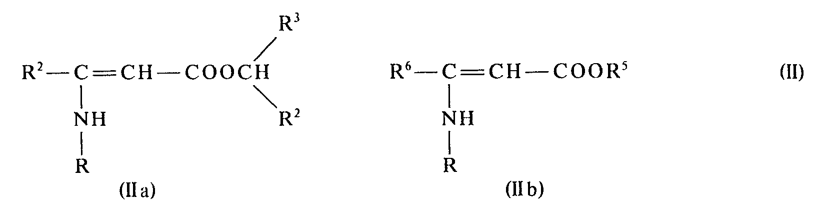 dans laquelle
R, R¹, R², R³, R⁵ et R⁶ ont la signification indiquée ci-dessus,
avec une mole d'ester d'acide β-cétocarboxylique de formule générale III 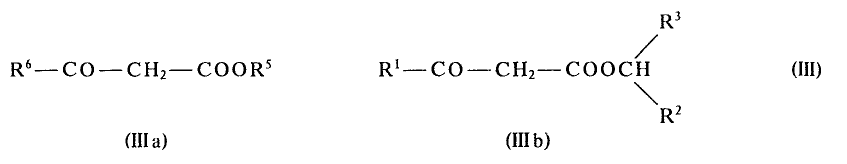 dans laquelle
R¹, R², R³, R⁵ et R⁶ ont la signification indiquée ci-dessus,
avec une mole d'aldéhyde de formule générale (IV) 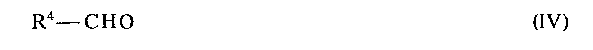 dans laquelle
R⁴ possède la signification indiquée ci-dessus,
éventuellement après isolation des composés ylidène se formant à partir de (III) et de (IV), de formule générale (V): 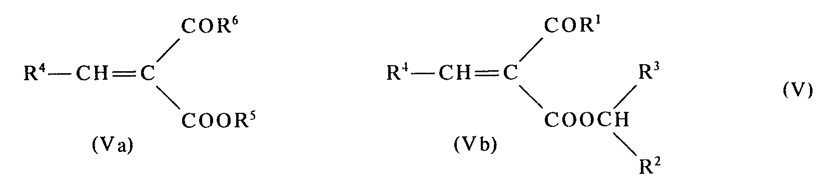 dans laquelle
R¹, R², R³, R⁴, R⁵ et R⁶ possèdent la signification indiquée ci-dessus,
éventuellement en présence d'eau ou de solvants organiques inertes à des températures entre 20 et 150° C.
